# EUROPEAN PATENT APPLICATION

(11) **EP 1 749 528 A1**
(43) Date of publication of application: **07.02.2007**
(21) Application number: 05291689.7
(22) Date of filing: 05.08.2005
(51) Int. Cl.: A61K 31/445, A61K 31/04, A61K 31/416, A61P 17/00

(54) **Pharmaceutical combinations containing a mu opioid agonist and an inhibitor of NO production**

(71) Applicant: Pharma C S.A., 59120 Loos (FR)
(72) Inventor: The designation of the inventor has not yet been filed
(74) Representative: Grünecker, Kinkeldey, Stockmair & Schwanhäusser Anwaltssozietät

(57) **Abstract**

The present invention relates to compositions and methods for treatment and/or prevention of oedema, pruritus and hyperalgesia often associated with activation of peripheral sensory nerves (neurogenic inflammation). The compositions, which may be formulated for topical and local administration, contain relatively selective µ-opioid agonists that are substantially devoid of central nervous system effects, in combination with inhibitors of NO production, preferably, but not limited to, inhibitors of nitric oxide synthase (NOS), and in particular non-selective inhibitors, or relatively selective inhibitors of the neuronal NOS (nNOS) and inducible NOS (iNOS) isoforms.

## Description

### 1.1 Field of the present invention

The present invention relates to compositions and methods for treatment and/or prevention of oedema, pruritus and hyperalgesia often associated with activation of peripheral sensory nerves (neurogenic inflammation). The compositions, which may be formulated for topical and local administration, contain relatively selective mu(µ)-opioid agonists that are substantially devoid of central nervous system effects, in combination with inhibitors of NO production, preferably, but not limited to, inhibitors of nitric oxide synthase (NOS), and in particular non-selective inhibitors, or relatively selective inhibitors of the neuronal NOS (nNOS) and inducible NOS (iNOS) isoforms. The latter agent permits the effective chronic use of the former agent, which when used alone is associated with the rapid development of tolerance, tachyphylaxis and diminution of therapeutic effectiveness.

More precisely, the local or topical application of a peripherally-restricted µ-opioid receptor agonist in combination with an inhibitor of nitric oxide (NO) production aims at the treatment of chronic pathological inflammatory and hyperalgesic conditions and associated symptoms (oedema, pain, pruritus etc.).

In the context of the present invention the use of the NO inhibitor allows the reduction or inhibition of side effects including the tolerance, associated with the single administration of µ receptor agonists.

### 1.2 Description of prior art

### Sensory nerves

Sensory nerves are found at body surfaces that are in direct contact with the external environment such as the skin, respiratory and gastrointestinal tracts and the bladder. Being at the interface between the internal and external environments, sensory nerves are in a unique position to monitor events occurring at the body surface and to initiate the appropriate actions necessary to maintain underlying tissue integrity. They protect the tissues from injury by irritants and infections by liberating a variety of neuropeptide transmitters (substance P (SP), calcitonin gene related peptide (CGRP), neurokinin A (NKA), neurokinin B (NKB) etc.) and recruiting the autonomic nervous system (parasympathetic (PNS) and sympathetic (SNS) nerves) to regulate epithelial, vascular, glandular and smooth muscle functions.
There are two types of sensory fibres, A-delta fibres which are myelinated and mediate the initial sharp pain response to noxious stimuli, and C-fibres which are non-myelinated and mediate the delayed, dull, prolonged pain response. These nociceptors have bare nerve endings and form a primitive sensory apparatus, reacting to heat, acid and inflammatory mediators released after mucosal injury or following mast cell degranulation.
Once released, sensory neuropeptides act upon specific receptors on target cells to initiate and amplify local mucosal vasodilatation, vascular permeability and glandular exocytosis and may facilitate vascular wall leukocyte adhesion and diapedesis. These neuropeptides mainly include the tachykinin, SP, which mediates bronchoconstriction and pro-inflammatory effects and CGRP which mediates vasodilatation and oedema. The central reception of nociceptive impulses in the brainstem and higher centres leads to appreciation of sensations like itch, burning, pain and congestion and initiates important reflexes such as sneeze, cough and the secretory reflexes via the PNS.

### Prejunctional modulation of sensory nerves

Direct modulation of the activity of sensory nerves inhibits certain pathological effects (rhinitis, cough, airways hyperreactivity, pain and hyperalgesia) arising from both central and peripheral reflexes and may have advantages over other therapeutic targets.
There are several known main pathways modulating sensory nerve activity some acting in a positive sense to increase the liberation of neuropeptides and others in an inhibitory fashion. A diverse range of chemical mediators that are either produced or released locally following tissue injury and inflammation can activate peripheral sensory nerve endings. These can either directly activate the sensory nerves (e.g. H⁺, ATP, glutamate, 5-HT, histamine, bradykinin, sensitize the nerve endings to the action of other stimuli (e.g. prostaglandins, prostacyclin, cytokines, IL-1β, IL-2, IL-6, IL-8, TNFα)) or exert regulatory effects on the sensory neurone, adjacent inflammatory cells and sympathetic nerves (e.g. bradykinin, tachykinins, NGF). Some agents that activate sensory nerves do so by acting directly on ion channels (e.g. H⁺ via acid sensitive ion channels, ATP via P2X receptors), whereas other agents sensitize sensory neurons by acting on G-protein coupled receptors (GPCRs) to alter intracellular messengers (e.g. cyclic AMP, Ca²⁺, inositol phosphate) and some of these activate protein kinases that phosphorylate ion channels and modulate their function.

With respect to prejunctional GPCRs, studies have shown that the e-NANC bronchoconstriction induced by electrical stimulation of the airways wall can be inhibited by activation of µ-opioid, GABA_{B}, α2-adrenoceptors and NPY receptors, thus implying an inhibition of neurotransmitter release from the C-fibre endings.
There is a precedent for targeting sensory nerves therapeutically in the form of the triptans which are used clinically for the symptomatic treatment of migraine. It is postulated that triptans stimulate 5-HT_{1B/1D} receptors on trigeminal nerve endings in the CNS and thereby reduce the release of vasoactive neurotransmitters from sensory nerve endings to relieve the migraine attack.

### Prejunctional opioid receptors on sensory nerves

Opioid receptors are present on the peripheral terminals of thinly myelinated and unmyelinated cutaneous sensory nerve fibers. Peripheral opioid effects are less evident in normal compared to inflamed tissues probably due to inflammation-induced disruption of the perineural barrier that normally limits access of these drugs to the nerve. Whilst inflammation enhances opioid receptor expression and transport to peripheral nerve terminals this process takes days and the initial upregulated response to opioids precedes these changes (minutes to hours).

µ-Opioid receptor subtype agonists are generally the most potent at producing peripheral analgesia. The effect results from stimulation of prejunctional µ-opioid receptors on sensory nerve endings (including the nociceptors) which via activation of a G-protein (Gi and/or Go) leads to a decrease in cyclic AMP, an increased K+ efflux and decreased Ca2+ entry into the nerve terminals. This attenuates the excitability of the peripheral nerve terminals, the propagation of action potentials and the release of inflammatory neuropeptides.

Inflammation due to the stimulation of sensory nerves (C- fibres) can also be modulated by relatively selective agonists of the µ-opioid receptor, for example loperamide, loperamide N oxide and their derivatives. Clearly a compound like loperamide, able to inhibit the liberation of neurotransmitters from sensory nerve endings should be more effective than postjunctional antagonists that target the individual neurotransmitter receptors. Several postjunctional antagonists, used in combination, would be needed to inhibit, for example, the effects of SP, CGRP and GRP and NKB.

### Peripheral opioid receptor agonists

A number of studies have shown the effectiveness of peripheral opioid mechanisms in the therapeutic setting. For example, morphine has been given via the intra-articular route during knee surgery and locally in dental surgery with positive results in terms of both efficacy, duration of action and minimal side effects. The topical route has also been employed acutely with morphine for the treatment of painful leg ulcers and skin lesions, burns and cutaneous pain syndromes. Nevertheless there is a general reluctance to use morphine even topically because of the highly addictive properties of the drug and the fact that a small proportion of even topically applied morphine has been shown to penetrate systemically.

Loperamide is an opioid agonist used for the treatment of diarrhoea, which is not bioavailable systemically, and yet has activity when injected locally or applied topically. There are several claims in the patent literature for the use of loperamide locally by injection into the affected site since its physicochemical properties act to limit its distribution in the body to outside the brain, thus reducing the propensity for CNS side effects. Clearly such an agent, if effective, would be theoretically superior to morphine since it would be less likely to produce dependence and other peripheral systemic side effects such as constipation. Preclinical studies have demonstrated that the topical administration of loperamide can produce effective anti-hyperalgesic effects in the rat hindpaw in a thermal injury model and in another study immersing the tails of mice in solutions of the solvent DMSO or DMSO plus morphine or DAMGO (a µ-opioid agonist). Further studies also demonstrated a peripheral action with agonists of the µ-opioid receptor. Indeed morphine and µ-opioid agonists like loperamide have figured in several patent claims as peripheral anti-hyperalgesic agents via local applications.

However, all opioid agonists provoke the rapid generation of tolerance and tachyphylaxis to their beneficial effects of stimulating prejunctional opioid receptors on sensory nerves. Their analgesic and anti-inflammatory effects are rapidly lost, making chronic therapy with such agents practically impossible. For example, repeated administration produces tolerance to the peripheral analgesic effects of morphine and the relatively selective µ-opioid agonist DAMGO and similar studies have shown that the peripheral analgesia induced by morphine is rapidly lost upon repeated injection. Thus peripheral opioid induced anti-hyperalgesia exhibits tolerance, similar to that observed with these agents when administered via systemic routes (e.g. spinal injection or oral).

US5994372 claims compositions, and methods using the compositions, for treatment of peripheral hyperalgesia where compositions contain an anti-hyperalgesia effective amount of one or more compounds that directly or indirectly interact with peripheral opiate receptors but that do not, upon topical or local administration, elicit substantial central nervous system effects. Loperamide is cited as one of the preferred compounds for use in the compositions and methods.

US6166039 and CA2229814 also claim methods for the topical or local treatment of peripheral hyperalgesia with compounds either acting directly or indirectly with opiate receptors and loperamide is again cited as a preferred molecule.

US5855907 claims a method of treatment for migraine using migraine-ameliorating effective amounts of loperamide hydrochloride via topical administration.

US6509028, US2002192288, WO200200195, US20020113331, US2003124190, CA2413545 and EP1296650 claim methods and compositions for treating pain of the mucous membranes by topical application of an opioid agent (morphine and loperamide cited) and/or a local anaesthetic agent.

There are several patents describing percutaneous delivery systems for opioid analgesics, for example US6355657 describes such a system and US2003215496, US2003064097 and US2002012680 describe compositions and methods for improved delivery of hydrophobic active ingredients, including loperamide and its derivatives.

Formulations for such topical applications include sprays, EP1150661, W0200045795, US2004213744, CA2359640, and film-forming compositions for coating the injured or inflamed site. CA2223514 and US5667773 disclose such formulations.

Clearly if a way could be found to prevent the development of tolerance this would result in a superior therapeutic composition.

### 1.3 Brief description of the invention

The present invention provides a new pharmaceutical composition containing at least one inhibitor of NO and at least one peripherally acting opioid, preferably a relatively selective, peripherally acting µ-opioid agonist like loperamide or its salts, oxides and derivatives. The new composition, preferably as a topical or local formulation not only increases the treatment time window, in that tolerance to the opioid was abolished or at least much delayed, thus permitting its effective use over a much longer time period, but also increases the therapeutic effectiveness of the opioid, permitting the utilisation of lower doses of this agent. Clearly this provides the potential for much more effective therapeutic intervention, especially in children, with minimal risk of side effects.

The composition as disclosed in the present invention may be used for the treatment of neurogenic inflammatory conditions. Treatment conditions include but are not limited to, inflammation following local infections, blisters, boils, or acute skin injuries, such as abrasions, burns, such as thermal, radiation, sunburn and chemical burns, windburn, frostbite, superficial cuts, surgical incisions, contusions, irritations, inflammatory skin conditions like pruritus, and including but not limited to poison ivy, and allergic rashes and dermatitis, insect stings and bites, joint inflammation, post-surgical hyperalgesic conditions and any condition that yields a hyperalgesic pain state are provided. Such conditions and indications, include, but are not limited to: a) skin conditions; b) oral, laryngeal and bronchial conditions and indications; c) ophthalmic indications and conditions; d) post surgical conditions and indications; e) recto-anal inflammations; and f) inflammations associated with infectious agents.

These treatment methods involve topical or local administration of compositions of the present invention that contain one or more compounds that exert anti-inflammatory activity via peripheral opiate receptors, but that do not exhibit CNS, CNS-mediated analgesic or systemic effects [particularly CNS effects at dosages at which they are topically or locally applied]. The intended locus of application includes, but is not limited to, any body surface or part that is amenable to local or topical treatment. Such body parts include, but are not limited to: the skin, joints, eyes, lips and mucosal membranes including internal mucus membranes.

The treatment methods use compositions containing opioid anti-diarrhoeal compounds or other opiate receptor agonist compounds that do not, upon topical or local administration, evoke CNS effects, as defined herein, particularly at the peripheral anti-inflammatory dosage. Compositions that contain the opioid anti-diarrhoeal compounds or other opiate receptor compounds are also provided.

Typically the compounds intended for use in the compositions and methods herein possess peripheral anti-inflammatory and anti-hyperalgesic activities and substantially no CNS activities, as defined herein, because, without being bound by any theory, they do not effectively cross the blood brain barrier. The failure to cross the blood brain barrier precludes the occurrence of the CNS systemic effects, so that there is limited potential for abuse. Other opioids, such as morphine, that readily cross the blood brain barrier could be effective as anti-inflammatories/anti-hyperalgesics, but their permeability through the blood brain barrier results in abuse liability.

In contrast, the compositions provided herein, contain opioids that do not, upon topical or local administration, substantially cross the blood brain barrier. The compounds intended for use in the methods and compositions provided herein include any compound that by virtue of its interaction, either directly or indirectly, with peripheral opioid receptors ameliorates the peripheral inflammatory/hyperalgesic state, but does not exhibit systemic CNS-mediated analgesic activity [i.e., analgesic activity by virtue of interaction with CNS opioid receptors] or CNS side-effects, including heaviness of the limbs, flush or pale complexion, clogged nasal and sinus passages, dizziness, depression, respiratory depression, sedation and constipation. These compounds include anti-diarrhoeals that act as anti-diarrhoeals via interaction with mu, delta or kappa receptors, especially µ opiate receptors agonists.

### Examples of such compounds include, but are not limited to the following:

Loperamide: [4 - (p - chlorophenyl) - 4 - hydroxyl -N - N -dimethyl - alpha.,.alpha.-diphenyl-1-piperidine butyramide hydrochloride], loperamide analogs and related compounds as defined in U.S. Pat. No. 3,884,916 and U.S. Pat. No. 3,714,159; U.S. Pat. No. 4,194,045, U.S. Pat. No. 4,116,963, U.S. Pat. No. 4,072,686, U.S. Pat. No. 4,069,223, U.S. Pat. No. 4,066,654, incorporated herein by reference, N-oxides of loperamide and analogues, metabolites and prodrugs thereof and related compounds as defined in U.S. Pat. No. 4,824,853, incorporated herein by reference, and related compounds, such as 4-(aroylamino)piperidine-butanamide derivatives and N-oxides thereof as defined in U.S. Pat. No. 4,990,521, incorporated herein by reference.

Because of its ready availability and demonstrated safety, loperamide oxide or loperamide HCl are most preferred.

The second main component of the composition as provided by the present invention is an inhibitor of NO production, i.e. a compound capable of inhibition NO production in a living body. Preferred inhibitors of NO production are NOS inhibitors, such as non-selective NOS inhibitors such as N-nitro-L-arginine, N(G)-nitro-L-arginine methyl ester and N(G)-monomethyl-L-arginine, the relatively selective inhibitors of the inducible NOS (iNOS) isoform like, but not limited to, aminoguanidine and the relatively selective nNOS isoform inhibitors such as, but not limited to, 7-nitroindazole.

Compositions formulated for topical and local administration for treatment and/or prevention of inflammation/hyperalgesia are also provided by the present invention. The compositions provided herein, may be formulated for single or multiple dosage administration, and contain an anti-inflammatory/anti-hyperalgesic effective amount (where the amount refers that which is delivered as a single dose) of one or more of first and second agents in a vehicle formulated for topical or local administration. Generally the compounds are provided in the form of a suspension or emulsion at concentrations of from about 0.1 %, preferably from greater than about 1 %, particularly when formulated in aqueous medium for application to the nasal passages or lungs, to up to 50% or more (by weight).

The compositions are formulated as creams, aqueous or non-aqueous suspensions, lotions, emulsions, suspensions or emulsions containing micronized particles, gels, foams, aerosols, solids and other suitable vehicles for application to the skin, eyes, lips and mucosa, as suppositories or creams for vaginal administration, and as combinations with bandages, patches, bioadhesives and dressings. The compounds may be formulated in combination with other agents, such as local anaesthetics, vasoconstrictors and other therapeutic agents. The other agents may be mixed in the compositions or provided separately and administered prior to, simultaneously with or subsequent to administration of the compositions provided for the methods herein. Such agents include, but are not limited to: antibiotics, including cephalosporins, beta.-lactams, tetracyclines, vancomycins, sulfas and aminoglycosides; antivirals, including acylovir; antifungals including clotrimazole; vasoconstrictors; non-steroidal anti-inflammatories (NSAIs) and steroids.

Methods of treating and/or preventing inflammation/hyperalgesia by applying an amount of the compositions provided herein effective to ameliorate or eliminate the inflammation/hyperalgesic state are provided. Thus, methods of treating and/or preventing pain and irritation associated with inflammation following local infection, blisters, boils, or acute skin injuries, such as abrasions, burns, superficial cuts, surgical incisions, toothaches, contusions, irritations, inflammatory skin conditions, including but not limited to poison ivy, and allergic rashes, pruritus and dermatitis and any condition that yields inflammation or a hyperalgesic pain state and other such conditions are provided by the present invention.

Articles of manufacture are also provided comprising packages containing the composition of the present invention and a label that indicates that the composition is to be used for treating and/or preventing inflammation/hyperalgesic conditions.

As used herein, topical application refers to application to the surface of the body, such as to the skin, eyes, mucosa and lips, which can be in or on any part of the body, including but not limited to the epidermis, any other dermis, or any other body tissue. Topical administration or application means the direct contact of the anti-inflammatory/anti-hyperalgesic with tissue, such as skin or membrane, particularly the cornea, or oral, vaginal or buccal mucosa. Topical administration also includes application to hardened tissue such as teeth and appendages of the skin such as nails and hair. Thus, for purposes herein topical application refers to application to the tissue of an accessible body surface, such as, for example, the skin (the outer integument or covering) and the mucosa (the mucus-producing, secreting and/or containing surfaces). In particular, topical application refers to applications that provide no or substantially no systemic delivery and/or systemic administration of the active compounds in the present compositions. Exemplary mucosal surfaces include the mucosal surfaces of the eyes, mouth (such as the lips, tongue, gums, cheeks, sublingual and roof of the mouth), larynx, oesophagus, bronchial, nasal passages, vagina and rectum/anus; in some embodiments, preferably the mouth, larynx, oesophagus, vagina and rectum/anus; in other embodiments, preferably the eyes, larynx, oesophagus, bronchial, nasal passages, vagina and rectum/anus; and in still other embodiments, preferably the vagina and rectum/anus.

A composition formulated for topical administration may be liquid or semi-solid (including, for example, a gel, lotion, emulsion, cream, ointment, spray or aerosol) or may be provided in combination with a "finite" carrier, for example, a non-spreading material that retains its form, including, for example, a patch, bioadhesive, dressing or bandage, it may be aqueous or non-aqueous; it may be formulated as a solution, emulsion, dispersion, a suspension or any other mixture.

As used herein, a composition refers to any mixture, including but not limited to, dispersions, emulsions, suspensions, and other mixtures. It may be a solution, a suspension, liquid, powder, a paste, aqueous, non-aqueous or any combination thereof.

As used herein, a combination refers to any association between two or among more items.

The combination as defined for the two agents as defined in claim 1 comprises combinations wherein each agent is formulated in isolation from the other agent, so that the mixing of the two agents takes only place upon application/aministration (kit-of-parts, comprising two formulations, one containing the first agent and one containing the second agent) as well as combinations wherein the two agents are present in one composition. The latter one is preferred. The disclosure in the present application with respect to specific formulations is intended to be valid however for both options mentioned above.

As used herein, a lack of (or without causing) CNS effects or systemic effects, including and particularly CNS effects and CNS-mediated effects, means that the agent preferably exhibits at least about 2-fold less activity in an assay or animal model for such effects than 2,2-diphenyl-4-[(4-carbethoxy-4-phenyl)piperidino]butyronitrile, generically known as diphenoxylate.

As used herein, the biological activity or bioactivity of a particular compound includes any activity induced, potentiated or influenced by the compound in vivo or in vitro. It also includes the abilities, such as the ability of certain molecules to bind to particular receptors and to induce a functional response. It may be assessed by in vivo assays or by in vitro assays, such as those exemplified herein.

As used herein, pharmaceutically acceptable salts, esters or other derivatives of the compounds include any salts, esters or derivatives that may be readily prepared by those of skill in this art using known methods for such derivatization and that produce compounds that may be administered to animals or humans without substantial toxic effects and that either are pharmaceutically active or are pro-drugs. For example, hydroxy groups can be esterified or etherified.
The therapeutic potential for peripherally acting compounds µ-opioid agonists like loperamide, capable of inhibiting sensory nerve activity, is large and includes certain inflammatory pain and skin conditions, irritable bowel syndromes (IBS), cough and rhinitis and migraine. All these potential indications would be amenable to treatment with topically administered agents. An example of the effectiveness and therapeutic utility of such preparations is given below for inflammatory pain conditions.

### Formulations and dosages

Typically therapeutically effective doses of the µ-opioid agonist, preferably loperamide as described earlier, formulated together with an inhibitor of NOS, hereafter designated as the **combination,** is made up to contain concentrations [by weight] of between 0.1 % up to about 50% or more of each of the two active ingredients, and preferably more than 1 % of each of them, for application to the treated tissue. Preferred ranges are from 1 to 50%, from 1 to 30 %, from 1 to 20 %, from 1 to 15% as well as from 2 to 50%, from 2 to 30 %, from 2 to 20 %, from 2 to 15% as well as from 5 to 50%, from 5 to 30 %, from 5 to 20 %, from 5 to 15% (by weight). The active ingredients may be administered once or may be divided into a number of smaller doses and administered at intervals of time. The precise dose of the combination may vary but the preferred ratio of the active ingredients in the combination preferably will be fixed (opioid:NOS), and will usually be in the preferred ratio range of 1:1 to 1:10, preferably 1:1 to 1:5. The dose and duration of treatment with the combination will vary as a function of the tissue being treated and this means that the ratio will be determined empirically using known clinical testing protocols or by extrapolation from in vivo animal test data. It is also to be noted that concentrations and doses of the combination will, of course, vary with the galenic formulation and may also vary with the individual being treated (age, sex etc.) and that for any particular subject specific dosage regimens should be adjusted over time according to the individuals need, response and tolerance to the product. The concentration ranges for the combination given are, therefore, meant as examples and are not intended to limit the scope or application of the claimed combination product formulations.

The active compounds in the combination may be solubilised or suspended in micronised or other suitable form or may be modified to produce more soluble products or to produce pro-drugs. The form of the final mixture will depend upon the intended route of administration and the solubility of the combination in the selected carriers or vehicles. The concentrations selected for the active ingredients in the combination will be adequate for treating hyperalgesic conditions associated with activation or sensitization of peripheral sensory nerves with minimal side effects and in many cases this will have to be determined empirically.

The combination of compounds will typically be in the range 0.001 % [by weight] or greater than 1% up to 50% or higher for both active ingredients and this concentration is higher than those effective via the systemic route for the active compounds. Preferable concentrations for both compounds by weight are greater than 1 % and up to 10% and aqueous suspensions and compositions will also contain 1 % or more of the compounds.

The resulting composition of the invention mixture may be a solution, suspension, emulsion or similar, formulated as creams, gels, ointments, emulsions, solutions, elixirs, lotions, suspensions, tinctures, pastes, foams, aerosols, irrigations, sprays, suppositories, bandages, or any other formulation suitable for topical or local administration.

The intended route of administration of the combination product described herein is topical or via local administration, and compositions are formulated in a manner suitable for each route of administration. Preferred modes of administration include topical application to the skin, eyes or mucosa, and local application to the joints, such as by intra-articular injection. Thus, typical vehicles are those suitable and available for pharmaceutical or cosmetic application to body surfaces or for local injection.

Pharmaceutical and cosmetic carriers or vehicles suitable for administration of the compounds provided herein include any such carriers known to those skilled in the art to be suitable for the particular mode of administration. The active compounds are included in the carrier in amounts sufficient to exert therapeutically useful effects in the absence of serious toxic effects on the treated individual. The effective concentration may be determined empirically by testing the compounds in in vivo systems or using standard clinical protocols.

For topical administration, the combination may be formulated in the form of gels, creams, lotions, solids, solutions or suspensions, or aerosols. Products tor treating human skin are formulated for topical application with an effective amount of the combination described herein, in effective concentration ranges [by weight], usually between about 0.1 % and 80%, preferably 0.1 to 50%, more preferably greater than about 1% up to about 50% or more in a cream, ointment, lotion, gel, solution or solid base or vehicle known in the art to be non-toxic and acceptable or suitable for application topically to the skin or mucous membranes. Aqueous suspensions are preferably formulated at concentrations greater than about 1 %, more preferably 2%.

The compositions provided herein, are preferably formulated for single dosage administration, and contain an effective amount of the two compounds in a vehicle formulated for topical or local administration. Generally the active compounds are provided in the form of a suspension or emulsion at concentrations from about 0.1%, preferably to greater than about 10%, particularly when formulated in aqueous medium for application to the nasal passages or airways.

The combination may be formulated as creams, aqueous or non-aqueous suspensions, lotions, emulsions, suspensions or emulsions containing micronised particles, gels, foams, aerosols, solids and other suitable vehicles for application to the skin, eyes, lips and mucosa, as suppositories or creams for vaginal administration, and as combinations with bandages, patches, bioadhesives and dressings. The combination may be formulated with other agents, such as local anesthetics and other therapeutic agents. The other agents may be mixed in the compositions are provided and administered prior to, simultaneously with or subsequent to administration of the compositions provided for the methods herein. Such agents include, but are not limited to: antibiotics, including cephalosporins, beta-lactams, tetracyclines, vancomycins, sulfas and aminoglycosides; antivirals, including acylovir; and antifungals including clotrimazole.

Methods of treating hyperalgesia by applying effective amounts of the combination product described to ameliorate or eliminate the hyperalgesic state are provided. Thus, methods of treating pain and irritation associated with inflammation following local infection, blisters, boils, or acute skin injuries, such as abrasions, burns, superficial cuts, surgical incisions, toothaches, contusions, irritations, inflammatory skin conditions, including but not limited to poison ivy, and allergic rashes and dermatitis and any condition that sensitizes or stimulates peripheral sensory nerve activity to produce a hyperalgesic pain state and other such conditions are provided.

As used herein, topical application refers to application to the surface of the body, such as to the skin, eyes, mucosa and lips, which can be in or on any part of the body, including but not limited to the epidermis, any other dermis, or any other body tissue. Topical administration or application means the direct contact of the anti-hyperalgesic with tissue, such as skin or membrane, particularly the cornea, or oral, vaginal or buccal mucosa. Topical administration also includes application to hardened tissue such as teeth and appendages of the skin such as nails and hair. A composition formulated for topical administration is generally liquid or semi-liquid carriers such a gel, lotion, emulsion, cream, plaster, or ointment, a spray or aerosol, or a "finite" carrier, i.e., a non-spreading substance that retains its form, such as a patch, bioadhesive, dressing and bandage or other device. It may be aqueous or non-aqueous; it may be formulated as a solution, emulsion or a suspension.

Local application includes intra-joint, such as intra-articular application, via injection, via catheter or delivery as part of a biocompatible device.

### Formulation of compositions for in vivo use and methods of use

Effective concentrations of the combination are mixed with a suitable pharmaceutical carrier or vehicle for topical or local administration. The product includes amounts of active ingredients effective at reducing hyperalgesia for which treatment is contemplated. The concentration of active compounds in the composition will depend on absorption, inactivation, excretion rates of the active compound, the dosage schedule, and amount administered as well as other factors known to those of skill in the art. Generally, the dosages are typically at least about 5 to 10 fold greater than the amount delivered when administered systemically, but this will depend on the galenic formulation and, if necessary, may be empirically determined from paradigms.

Pharmaceutical carriers or vehicles suitable for administration of the combination and for the methods provided herein include any such carriers known to those skilled in the art to be suitable for the particular mode of administration. In addition, the combination may be formulated as the sole pharmaceutically active ingredients in the composition or in certain cases may be combined with other active ingredients.

To formulate the composition of the combination, the weight fraction of each compound is dissolved, suspended, dispersed, or otherwise mixed in (a) selected compatible vehicle(s) and then combined to give final effective concentrations. Generally, emollient or lubricating vehicles that help hydrate the skin are more preferred than volatile vehicles, such as ethanol, that dry the skin. Examples of suitable bases or vehicles for preparing compositions for use with human skin are petrolatum, petrolatum plus volatile silicones, lanolin, cold cream [USP], and hydrophilic ointment [USP].

The choice of acceptable vehicles is largely determined by the mode of application and tissue to be treated. Suitable pharmaceutically acceptable vehicles for topical application include lotions, creams, solutions, gels, bandages, tapes and the like. Generally, the vehicle is organic in nature or an aqueous emulsion capable of having the selected combination of active compounds, which may be micronized, dispersed, suspended or dissolved within. The vehicle may include pharmaceutically-acceptable emollients, skin penetration enhancers, colouring agents, fragrances, emulsifiers, thickening agents, and solvents.

For local internal administration, such as intra-articular administration, the combination is preferably formulated as a suspension in an aqueous-based media such as isotonically buffered saline. It may also be combined with a biocompatible support or bioadhesive intended for internal administration.

### 1. Lotions

The lotions contain effective concentrations of the combination of compounds. The effective concentration is that able to deliver an anti-hyperalgesic amount, typically at a concentration of between about 0.1-50% [by weight] of the two compounds provided herein. The lotions also contain [by weight] from 1 % to 50%, preferably from 3% to 15%, of an emollient and the balance water, a suitable buffer, a C2 or C3 alcohol, or a mixture of water or the buffer and the alcohol. Any emollients known to those of skill in the art as suitable for application to human skin may be used. These include, but are not limited to, the following:
(a) Hydrocarbon oils and waxes, including mineral oil, petrolatum, paraffin, ceresin, ozokerite, microcrystalline wax, polyethylene, and perhydrosqualene.
(b) Silicone oils, including dimethylpolysiloxanes, methylphenylpolysiloxanes, watersoluble and alcohol-soluble silicone-glycol copolymers.
(c) Triglyceride fats and oils, including those derived from vegetable, animal and marine sources. Examples include, but are not limited to, castor oil, sunflower oil, cotton seed oil, corn oil, olive oil, cod liver oil, almond oil, avocado oil, palm oil, sesame oil, and soybean oil.
(d) Acetoglyceride esters, such as acetylated monoglycerides.
(e) Ethoxylated glycerides, such as ethoxylated glyceryl monstearate.
(f) Alkyl esters of fatty acids having 10 to 20 carbon atoms. Methyl, isopropyl and butyl esters of fatty acids are useful herein. Examples include, but are not limited to, hexyl laurate, isohexyl laurate, isohexyl palmitate, isopropyl palmitate, isopropyl myristate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate.
(g) Alkenyl esters of fatty acids having 10 to 20 carbon atoms. Examples thereof include, but are not limited to, oleyl myristate, oleyl stearate, and oleyl oleate.
(h) Fatty acids having 9 to 22 carbon atoms. Suitable examples include, but are not limited to, pelargonic, lauric, myristic, palmitic, stearic, isostearic, hydroxystearic, oleic, linoleic, ricinoleic, arachidonic, behenic, and erucic acids.
(i) Fatty alcohols having 10 to 22 carbon atoms, such as, but not limited to, lauryl, myristyl, cetyl, hexadecyl, stearyl, isostearyl, hydroxystearyl, oleyl, ricinoleyl, behenyl, erucyl, and 2-octyl dodecyl alcohols.
(j) Fatty alcohol ethers, including, but not limited to ethoxylated fatty alcohols of 10 to 20 carbon atoms, such as, but are not limited to, the lauryl, cetyl, stearyl, isostearyl, oleyl, and cholesterol alcohols having attached thereto from 1 to 50 ethylene oxide groups or 1 to 50 propylene oxide groups or mixtures thereof.
(k) Ether-esters, such as fatty acid esters of ethoxylated fatty alcohols.
(l) Lanolin and derivatives, including, but not limited to, lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin, acetylated lanolin alcohols, lanolin alcohols linoleate, lanolin alcohols ricinoleate, acetate of lanolin alcohols ricinoleate, acetate of ethoxylated alcohols-esters, hydrogenolysis of lanolin, ethoxylated hydrogenated lanolin, ethoxylated sorbitol lanolin, and liquid and semisolid lanolin absorption bases.
(m) Polyhydric alcohols and polyether derivatives, including, but not limited to, propylene glycol, dipropylene glycol, polypropylene glycol [M.W. 2000-4000], polyoxyethylene polyoxypropylene glycols, polyoxypropylene polyoxyethylene glycols, glycerol, ethoxylated glycerol, propoxylated glycerol, sorbitol, ethoxylated sorbitol, hydroxypropyl sorbitol, polyethylene glycol [M.W. 200-6000], methoxy polyethylene glycols 350, 550, 750, 2000, 5000, poly(ethylene oxide) homopolymers [M.W. 100,000-5,000,000], polyalkylene glycols and derivatives, hexylene glycol (2-methyl-2,4-pentanediol), 1,3-butylene glycol, 1,2,6,-hexanetriol, ethohexadiol USP (2-ethyl-1,3-hexanediol), C15 -C18 vicinal glycol and polyoxypropylene derivatives of trimethylolpropane.
(n) Polyhydric alcohol esters, including, but not limited to, ethylene glycol mono- and di-fatty acid esters, diethylene glycol mono- and di-fatty acid esters, polyethylene glycol [M.W. 200-6000], mono- and di-fatty esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.
(o) Wax esters, including, but not limited to, beeswax, spermaceti, myristyl myristate, and stearyl stearate and beeswax derivatives, including, but not limited to, polyoxyethylene sorbitol beeswax, which are reaction products of beeswax with ethoxylated sorbitol of varying ethylene oxide content that form a mixture of ether-esters.
(p) Vegetable waxes, including, but not limited to, carnauba and candelilla waxes.
(q) Phospholipids, such as lecithin and derivatives.
(r) Sterols, including, but not limited to, cholesterol and cholesterol fatty acid esters.
(s) Amides, such as fatty acid amides, ethoxylated fatty acid amides, and solid fatty acid alkanolamides.

The lotions further preferably contain [by weight] from 1 % to 10%, more preferably from 2% to 5%, of an emulsifier. The emulsifiers can be nonionic, anionic or cationic. Examples of satisfactory nonionic emulsifiers include, but are not limited to, fatty alcohols having 10 to 20 carbon atoms, fatty alcohols having 10 to 20 carbon atoms condensed with 2 to 20 moles of ethylene oxide or propylene oxide, alkyl phenols with 6 to 12 carbon atoms in the alkyl chain condensed with 2 to 20 moles of ethylene oxide, mono- and di-fatty acid esters of ethylene oxide, mono- and di-fatty acid esters of ethylene glycol wherein the fatty acid moiety contains from 10 to 20 carbon atoms, diethylene glycol, polyethylene glycols of molecular weight 200 to 6000, propylene glycols of molecular weight 200 to 3000, glycerol, sorbitol, sorbitan, polyoxyethylene sorbitol, polyoxyethylene sorbitan and hydrophilic wax esters. Suitable anionic emulsifiers includ, but are not limited to, the fatty acid soaps, e.g. sodium, potassium and triethanolamine soaps, wherein the fatty acid moiety contains from 10 to 20 carbon atoms. Other suitable anionic emulsifiers include, but are not limited to, the alkali metal, ammonium or substituted ammonium alkyl sulfates, alkyl arylsulfonates, and alkyl ethoxy ether sulfonates having 10 to 30 carbon atoms in the alkyl moiety. The alkyl ethoxy ether sulfonates contain from 1 to 50 ethylene oxide units. Among satisfactory cationic emulsifiers are quaternary ammonium, morpholinium and pyridinium compounds. Certain of the emollients described in preceding paragraphs also have emulsifying properties. When a lotion is formulated containing such an emollient, an additional emulsifier is not needed, though it can be included in the composition.

The balance of the lotion is water or a C2 or C3 alcohol, or a mixture of water and the alcohol. The lotions are formulated by simply admixing all of the components together. The combination of compounds, preferably but not limited to, for example, loperamide and L-NAME, are dissolved, suspended or otherwise uniformly dispersed, in the mixture.

Other conventional components of such lotions may be included. One such additive is a thickening agent at a level from 1% to 10% by weight of the composition. Examples of suitable thickening agents include, but are not limited to: cross-linked carboxypolymethylene polymers, ethyl cellulose, polyethylene glycols, gum tragacanth, gum kharaya, xanthan gums and bentonite, hydroxyethyl cellulose, and hydroxypropyl cellulose.

### 2. Creams

The creams are formulated to contain concentrations able to deliver an anti-hyperaigesic effective amount of the two compound to the treated tissue, typically at between about 0.1%, preferably at greater than 1 % up to and greater than 50%, preferably between about 3% and 50%, more preferably between about 5% and 15% of one ore more the compounds provided herein. The creams also contain from 5% to 50%, preferably from 10% to 25%, of an emollient and the remainder is water or other suitable non-toxic carrier, such as an isotonic buffer. The emollients, as described above for the lotions, can also be used in the cream compositions. The cream may also contain a suitable emulsifier, as described above. The emulsifier is included is in the composition at a level from 3% to 50%, preferably from 5% to 20%.

### 3. Solutions and suspensions for topical and local administration

The solutions are formulated to contain amounts of the combination able to exert an anti-hyperalgesic activity, typically at a concentration [by weight] of between about 0.1-50%, preferably at least more than 1 %, more preferably more than 2%, of the two compounds provided herein. The balance is water, a suitable organic solvent or other suitable solvent or buffer. Suitable organic materials useful as the solvent or a part of a solvent system are as follows: propylene glycol, polyethylene glycol [M.W. 200-600], polypropylene glycol [M.W. 425-2025], glycerine, sorbitol esters, 1,2,6-hexanetriol, ethanol, isopropanol, diethyl tartrate, butanediol, and mixtures thereof. Such solvent systems can also contain water.

Solutions or suspensions used for local application can include any of the following components: a sterile diluent, such as water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid [EDTA]; buffers, such as acetates, citrates and phosphates; and agents for the adjustment of tonicity such as sodium chloride or dextrose. Liquid preparations can be enclosed in ampules, disposable syringes or multiple dose vials made of glass, plastic or other suitable material. Suitable carriers may include physiological saline or phosphate buffered saline [PBS], and the suspensions and solutions may contain thickening and solubilizing agents, such as glucose, polyethylene glycol, and polypropylene glycol and mixtures thereof. Liposomal suspensions, may also be suitable as pharmaceutically acceptable carriers. These may be prepared according to methods known to those skilled in the art.

These compositions that are formulated as solutions or suspensions may be applied to the skin, or, may be formulated as an aerosol or foam and applied to the skin as a spray-on. The aerosol compositions typically contain [by weight] from 25% to 80%, preferably from 30% to 50%, of a suitable propellant. Examples of such propellants are the chlorinated, fluorinated and chlorofluorinated lower molecular weight hydrocarbons. Nitrous oxide, carbon dioxide, butane, and propane are also used as propellant gases. These propellants are used as understood in the art in a quantity and under a pressure suitable to expel the contents of the container.

Suitably prepared solutions and suspensions may also be topically applied to the eyes and mucosa. Solutions, particularly those intended for ophthalmic use, may be formulated as 0.01 %-10% isotonic solutions, pH about 5-7, with appropriate salts, and preferably containing one or more of the compounds herein at a concentration of about 0.1 %, preferably greater than 1 %, up to 50% or more. Suitable ophthalmic solutions are known which describes typical compositions of ophthalmic irrigation solutions and solutions for topical application. Such solutions, which have a pH adjusted to about 7.4, contain, for example, 90-100 mM sodium chloride, 4-6 mM dibasic potassium phosphate, 4-6 mM dibasic sodium phosphate, 8-12 mM sodium citrate, 0.5-1.5 mM magnesium chloride, 1.5-2.5 mM calcium chloride, 15-25 mM sodium acetate, 10-20 mM D.L.-sodium .beta.-hydroxybutyrate and 5-5.5 mM glucose.

The active combination can also be mixed with further active materials, that do not impair their desired action, or with materials that supplement the desired action, including viscoelastic materials, such as hyaluronic acid, methyl-cellulose, methyl hyaluronate, polyacrylamide and polymethacrylamide. These viscoelastic materials are present generally in amounts ranging from about 0.5 to 5.0%, preferably 1 to 3% by weight of the conjugate material and serve to coat and protect the treated tissues. The compositions may also include a dye, such as methylene blue or other inert dye, so that the composition can be seen when injected into the eye or contacted with the surgical site during surgery.

### 4. Gels

Gel compositions can be formulated by simply admixing a suitable thickening agent to the previously described solution or suspension compositions. Examples of suitable thickening agents have been previously described with respect to the lotions.

The gelled compositions contain an effective amount of the combination to treat hyperalgesic conditions, typically at a concentration of between about 0.1-50% by weight or more of one or more of the compounds provided herein.; from 5% to 75%, preferably from 10% to 50%, of an organic solvent as previously described; from 0.5% to 20%, preferably from 1% to 10% of the thickening agent; the balance being water or other aqueous carrier.

### 5. Solids

Compositions of solid forms may be formulated as stick-type compositions intended for application to the lips or other parts of the body. Such compositions contain an effective amount of the combination of compounds herein described. The amount is typically an amount effective to deliver an anti-hyperyperalgesic amount, typically at a concentration of between about 0.1-50% or more of the combination. The solids also contain from about 40% to 98%, preferably from about 50% to 90%, of the previously described emollients. This composition can further contain from 1% to 20%, preferably from 5% to 15%, of a suitable thickening agent, and, if desired or needed, emulsifiers and water or buffers. Thickening agents previously described with respect to lotions are suitably employed in the compositions in solid form.

Other ingredients, such as preservatives, including methyl-paraben or ethyl-paraben, perfumes, dyes or the like, that are known in the art to provide desirable stability, fragrance or color, or other desirable properties, such as shielding from actinic rays from the sun, to compositions for application to the skin may also be employed in compositions for such topical applications.

### 6. Additional ingredients

Other active ingredients, include, but are not limited to antibiotics, antivirals, antifungals, anti-inflammatories, including steroidal and non-steroidal anti-inflammatories, anesthetics and mixtures thereof.

### E. Articles of manufacture

The compounds and compositions provided herein may be packaged as articles of manufacture containing packaging material, one or more of the compounds provided herein, which is effective for ameliorating peripheral hyperalgesia, within the packaging material, and a label that indicates that the compound, N-oxide, acid, salt or other derivative thereof is used for treating hyperalgesic conditions.

### F. Methods of treatment

Compositions for use with human skin preferably may be applied at least once per day or, if necessary to achieve the desired result, more often, to the areas of the skin for which treatment is sought. It is understood that the precise treatment regimen depends upon the individual treated and may be ascertained empirically depending upon the formulation and, particularly, the age of the treated individual. Any regimen is acceptable as long as the desired anti-hyperalgesic effects are achieved without substantial deleterious or sustained undesirable side effects.

The methods for treating human skin are practiced by applying to the skin, preferably at least daily, a composition suitable for human skin treatment or treatment of mucosal membranes and other body surface tissues, including the vagina, rectum, mouth, eyes and other such tissues. The compositions may be injected into joints or other inflamed areas.

Compositions may also be combined with bandages, bioadhesives and other dressings and devices and applied to the body in combination therewith.

## Claims

1. A pharmaceutical combination comprising
a) a first agent selected from peripherally acting µ opioid agonists, and
b) a second agent selected from an inhibitor of NO production.

2. The pharmaceutical combination according to claim 1 wherein the first agent is selected among compounds having a relatively selective interactions with µ opioid receptors and not being bioavailable systematically, which preferably do not substantially cross blood brain barrier.

3. The pharmaceutical combination according to claims 1 or 2 wherein the first agent is selescted from loperamide and loperamide derivatives.

4. The pharmaceutical combination according to any of claims 1 to 3 wherein the first agent is selected from loperamide HCl and loperamide oxide.

5. The pharmaceutical combination according to any of claims 1 to 4 wherein the first agent is present in an amount of from 0.001 to 50 wt.%.

6. The pharmaceutical combination according to any of claims 1 to 5 wherein the second agent is selected from inhibitors of NOS.

7. The pharmaceutical combination of any of claims 1 to 6, wherein the second agent is selected from non-selective NOS inhibitors such as N-nitro-L-arginine, N(G)-nitro-L-arginine methyl ester and N(G)-monomethyl-L-arginine, the relatively selective inhibitors of the inducible NOS (iNOS) isoform, such as aminoguanidine and the relatively selective nNOS isoform inhibitors such as 7-nitroindazole.

8. The pharmaceutical combination according to any of claims 1 to 7 wherein the second agent is present in an amount of from 0.001 to 50 wt.%.

9. The pharmaceutical combination according to any of claims 1 to 8 for ameliorating the peripheral inflammatory/hyperalgesic state, while not exhibiting systemic CNS-mediated analgesic activity [i.e., analgesic activity by virtue of interaction with CNS opioid receptors] or CNS side-effects, including heaviness of the limbs, flush or pale complexion, clogged nasal and sinus passages, dizziness, depression, respiratory depression, sedation and constipation.

10. The pharmaceutical combination according to any of claims 1 to 9 wherein the first agent is selected from loperamide and derivatives thereof and the second agent is selected among N-nitro-L-arginine, N(G)-nitro-L-arginine methyl ester, N(G)-monomethyl-L-arginine, aminoguanidine and 7-nitroindazole.

11. The pharmaceutical combination according to any of claims 1 to 10 for use in the preparation of a medicament for the treatment and/or prevention of neurogenic inflammatory conditions, including inflammation following local infections, blisters, boils, or acute skin injuries, such as abrasions, burns, such as thermal, radiation, sunburn and chemical burns, windburn, frostbite, superficial cuts, surgical incisions, contusions, irritations, inflammatory skin conditions like pruritus, and including but not limited to poison ivy, and allergic rashes and dermatitis, insect stings and bites, joint inflammation, post-surgical hyperalgesic conditions and any condition that yields a hyperalgesic pain state, including a) skin conditions; b) oral, laryngeal and bronchial conditions and indications; c) ophthalmic indications and conditions; d) post surgical conditions and indications; e) gastrointestinal and recto-anal inflammations; and f) inflammations associated with infectious agents.

12. The pharmaceutical combination according to any of claims 1 to 11 in the form of a solution, suspension, emulsion, dry powder or aerosol, formulated as cream, gel, ointment, emulsion, solution, elixir, lotion, suspension, tincture, paste, foam, aerosol, irrigation, spray, suppository, bandage, or any other formulation suitable for topical or local administration.

13. A process for preparing the pharmaceutical composition of claims 1 to 12 comprising the step of combining a therapeutically effective amount of a first and a second agent with a pharmaceutically acceptable carrier

14. A method of treating a patient suffering from inflammation following local infections, blisters, boils, or acute skin injuries, such as abrasions, burns, such as thermal, radiation, sunburn and chemical burns, windburn, frostbite, superficial cuts, surgical incisions, contusions, irritations, inflammatory skin conditions like pruritus, and including poison ivy, and allergic rashes and dermatitis, insect stings and bites, joint inflammation, post-surgical hyperalgesic conditions and any condition that yields a hyperalgesic pain state, comprising administering a therapeutically effective amount of the pharmaceutical composition of any of claims 1 to 12 to a patient in need thereof.

15. A method of treating a patient suffering from a) skin conditions; b) oral, laryngeal and bronchial conditions and indications; c) ophthalmic indications and conditions; d) post surgical conditions and indications; e) gastrointestinal and recto-anal inflammations; and f) inflammations associated with infectious agents, comprising administering a therapeutically effective amount of the pharmaceutical composition of any of claims 1 to 12 to a patient in need thereof.

16. A method of reducing the tolerance to the therapeutic effects of peripherally acting µ opioid agonists by administering an agent inhibiting NO production, preferably an inhibitors of NOS.

17. The method according to any of claims 14 or 15 wherein the administration is effected topically or locally.
